# EUROPEAN PATENT SPECIFICATION

(11) **EP 1 085 102 B1**
(45) Date of publication and mention of the grant of the patent: **18.07.2007**
(21) Application number: 00308156.9
(22) Date of filing: 19.09.2000
(51) Int. Cl.: C12Q 1/68

(54) **A method for reverse transcribing RNA isolated in dry form**
Methode zur reversen Transkription von im getrockneten Zustand isolierter RNA
Méthode d'une transcription inverse d'ARN isolé sous forme sèche

(30) Priority: 20.09.1999 US 398888
(43) Date of publication of application: 21.03.2001
(73) Proprietor: ORTHO-CLINICAL DIAGNOSTICS, INC., Rochester, NY 14650 (US)
(72) Inventor: Patterson, David R., San Diego, California 92128 (US); Puskas, John A., Rochester, New York 14613 (US); Song, Keming, Ballwin, Missouri 63021 (US)
(74) Representative: Fisher, Adrian John

(56) References cited:
- EP-A- 1 026 263
- EP-A- 1 035 220
- CUNNINGHAM C K ET AL: "Comparison of human immunodeficiency virus 1 DNA polymerase chain reaction and qualitative and quantitative RNA polymerase chain reaction in human immunodeficiency virus 1-exposed infants." THE PEDIATRIC INFECTIOUS DISEASE JOURNAL. UNITED STATES JAN 1999, vol. 18, no. 1, January 1999 (1999-01), pages 30-35, XP009004762 ISSN: 0891-3668
- NEDJAR S ET AL: "CO-AMPLIFICATION OF SPECIFIC SEQUENCES OF HCV AND HIV-1 GENOMES BY USING THE POLYMERASE CHAIN REACTION ASSAY: A POTENTIAL TOOL FOR THESIMULTANEOUS DETECTION OF HCV AND HIV-1" JOURNAL OF VIROLOGICAL METHODS, AMSTERDAM, NL, vol. 35, no. 3, 1991, pages 297-304, XP001004815 ISSN: 0166-0934
- GONZALEZ-VILLASENOR L I ET AL: "MULTIPLEX RT-PCR BASED TEST FOR HTLV-I/II, HIV-1, HCV AND HBV" CLINICAL CHEMISTRY, AMERICAN ASSOCIATION FOR CLINICAL CHEMISTRY. WINSTON, US, vol. 41, no. 11, 1995, page 9 XP001002366 ISSN: 0009-9147

## Description

### Field of the Invention

The present invention relates to improved methods for transcribing and amplifying nucleic acids. In particular, the present invention provides a method for reverse transcribing RNA by addition of an RNA transcription admixture directly to a dry composition comprising the RNA.

### Background of the Technology

When preparing a sample comprising RNA for reverse transcription and amplification, the integrity of the RNA is of highest concern. The degradation of RNA by RNase before performing a reverse transcription reaction to synthesize cDNA from the RNA can be catastrophic. If any degradation of the RNA occurs, overall system sensitivity is lost for an assay using amplification technologies. If a false negative occurs due to RNA degradation, the result could be disastrous for a patient when testing for such infectious diseases as the human immunodeficiency virus (HIV) or the hepatitis C virus (HCV).

A number of different procedures are used for RNA sample preparation: including a guanidinium-based sample preparation method as described in Molecular Cloning: A Laboratory Manual by Sambrook, Fritsch, and Maniatis ((1989) Cold Spring Harbor Laboratory Press, New York), Gentra System's (Minneapolis, MN) PURESCRIPT RNA Isolation Kit, and the MONITOR HIV-RNA quantitative test kit of F. Hoffman La Roche.

After isolating the RNA, usually as a centrifuged pellet, using these or related methods, all previous protocols require rehydrating the pellet comprising the RNA in a volume at least of 20 µL of RNase-free water (diethyl pyrocarbonate treated water), with some procedures requiring rehydration in up to 400 µL of RNase-free water. After rehydrating the RNA, an aliquot of the solution containing the rehydrated RNA is removed and added to a separate RNase-free tube where reverse transcription and PCR are conducted. Thus, only a portion of the isolated RNA is used in subsequent reverse-transcription and polymerase chain reaction amplification.

EP-A-1026263 and EP-A-1035220, both unpublished at the priority date of the present application, disclose methods in which an RNA pellet is resuspended in a buffer mix prior to adding reverse transcription reagents.

### Summary of the Invention

The present invention eliminates the need for separate rehydration and transfer steps and enables the entire amount of isolated RNA to be used in subsequent procedures such as reverese-transcription and polymerase chain reaction. The present invention dramatically reduces the risk of RNA degradation by RNase and increases the overall sensitivity of subsequent reverese-transcription and polymerase chain reaction (RT-PCR) steps. Isolation of RNA in dry form from a sample can be carried out using for example guanidinium-based methods, a PURESCRIPT RNA Isolation method, or similar methods known in the art. The method of the invention is particularly advantageous when used with samples having a low copy number target RNA. A low copy number is defined, for the purposes of the present invention, as less than or equal to about 250 copies of the target RNA per milliliter of sample. For example, samples obtained from individuals afflicted with HIV or HCV who are in the early stages of infection prior to seroconversion will generally contain a low viral or viral nucleic acid titer.

It is an object of the present invention to provide a method for reverse transcribing an RNA target. The method comprising the steps of:
(i) isolating nucleic acids from a sample to form a dry composition comprising a target RNA; and
(ii) contacting the dry composition directly with an RNA transcription admixture comprising a primer that will anneal to the RNA target, four different nucleoside triphosphates, and an enzyme having reverse transcriptase activity, under conditions such that the RNA target is reverse transcribed to form cDNA complementary to the RNA target.

Subsequent to reverse-transcription, the cDNA, thus formed, can be used in nucleic acid amplification methods such as the polymerase chain reaction (PCR), strand displacement amplification (SDA), ligase chain reaction (LCR) and others known in the art.

Details of PCR are well known. In summary, two or more oligonucleotide primers are annealed to the denatured strands of a target nucleic acid, and primer extension products are formed by polymerization of deoxynucleoside triphosphates by a polymerization agent to form a complementary strand. The polymerization agent is often a thermostable DNA polymerase. A typical PCR protocol involves repetitive cycles of template nucleic acid denaturation, primer annealing and extension of the annealed primers by the polymerization agent, resulting in exponential amplification of the target nucleic acid. Hence, the detection of targets existing in very low concentrations in a sample becomes possible.

Various other objects and advantages will be apparent from the detailed description of the invention.

### Brief Description of the Drawings

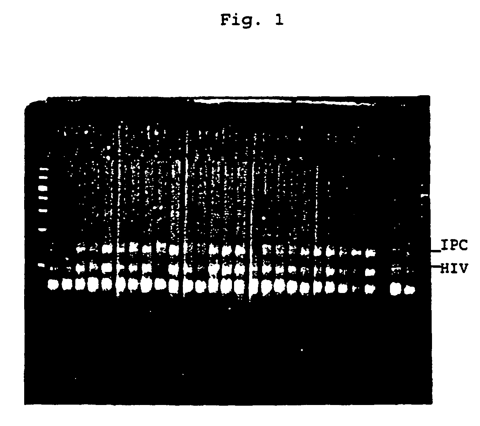

Figure 1 is a gel photograph showing the results of the experiments described in Example 1.

### Detailed Description of the Invention

The present invention provides improved methods for reverse transcribing and amplifying nucleic acids, including DNA and RNA. It provides a simple and efficient method for reverse transcribing RNA isolated from a sample in dry form. This method offers advantages over previously known procedures, as stated above, of increased sensitivity, decreased risk of degradation of RNA by RNase, elimination of separate rehydration and transfer steps, and it enables the entire amount of isolated RNA to be utilized.

In the method of the present invention, RNA is isolated from a sample and reverse transcribed to form cDNA. The RNA present in the sample is isolated using methods that are capable of producing a dry composition comprising the nucleic acid. Such methods include, but are not limited to, acid-phenol treatment (Perry, R.P. et al (1972) Biochem. Biophys. Acta Vol 262, pg. 220, and RNAgents Total RNA Isolation System, Promega Corporation, Madison, WI), Qiagen RNeasy Kit for cells (Qiagen, Germany), Qiagen RNeasy Kit for plasma (Qiagen, Germany), Bio 101 RNeasy Kit for cells (Bio101, San Diego, CA), guanidine-HCl treatment (Molecular Cloning: A Laboratory Manual 2nd Edition, Sambrook, Fritsch, Maniatis) 1989 Cold Spring Harbor Laboratory Press, New York)), PURESCRIPT RNA Isolation Kit (Gentra Systems,Inc., Minneapolis, MN), and differential precipitation methods (Birnboim, Nucleic Acid Research, Vol 16:4, pages 1487-1497, 2-25-1988).

When the dry nucleic acid composition has been obtained, according to the present invention, it can be combined directly with a solution comprising an enzyme having reverse transcriptase activity, thereby eliminating separate rehydration and transfer steps required in previous methods, thereby utilizing the entire amount of RNA so obtained.

Enzymes suitable for use in the present invention include, but are not limited to, reverse transcriptases and DNA polymerases having reverse transcriptase activity. Preferably, the DNA polymerase is thermostable. Examples of suitable reverse transcriptases include, but are not limited to Superscript RNaseH-RT and Superscript II RNaseH-RT both from GibcoBRL (Gaithersburgh, MD) and AMV (Avian Myeloblastosis Virus) and M-MLV (Moloney Murine Leukemia Virus). Similarly, examples of suitable thermostable DNA polymerases include, but are not limited to, Tfl (Thermus flavus) DNA polymerase, Tli (Thermus litorlis) DNA polymerase, and Taq (Thermus aquaticus) Polymerase. An example of a DNA Polymerase having reverse transcriptase activity includes but is not limited to Tth (Thermus thermophilus) DNA Polymerase. All the above-cited enzymes are available from various commercial vendors.

In addition to an enzyme having reverse transcriptase activity, other reagents can be combined directly with the dry nucleic acid composition such as, a nucleotide primer that will anneal to the target RNA and four different nucleoside triphosphates (dNTPs). These reagents are combined with the dry nucleic acid composition under conditions such that the target RNA is reverse transcribed to form cDNA complementary to the target. Such conditions and details on reverse transcribing RNA are well known and provided in numerous publications, for example, U.S. Patent No. 5,322,770, Promega PCR Guide, Chapters 4-6 (Promega, Madison, WI)and GeneAmp EzrTth RNA PCR Kit Technical Manual (Perkin-Elmer Cat.No. BIO-71.

The term "primer" refers to an oligonucleotide, whether naturally occurring or synthetically produced, that is capable of acting as a point of initiation of synthesis when placed under conditions in which synthesis of a primer extension product complementary to the nucleic acid template is induced.

The present invention is suitable for transcribing and amplifying RNA from a number of sources. Such sources include, but are not limited to, biological samples, nucleic acid preparations from organisms (viral and bacterial), cellular debris, purified total RNA and purified mRNA. The term "biological sample" includes, but is not limited to, cellular or viral material, hair, body fluids or cellular material containing nucleic acids which can be detected.

The cDNA that is formed can be amplified using known techniques as stated above. The general principles and conditions for amplification and detection of nucleic acids, including cDNAs, using PCR are quite well known. Details for practicing PCR are provided in numerous publications including U.S. Patent Nos. 4,683,195; 4,683,202 and 4,965,188. Thus, in view of the teaching in the art and the specific teaching provided herein, a worker skilled in the art should have no difficulty in practicing the present invention by amplifying cDNA generated from RNA isolated in dry form and reverse transcribed according to the present invention.

### EXAMPLES

The examples that follow are provided to illustrate certain embodiments of the present invention, and are not to be construed as limiting the invention. The PURESCRIPT RNA isolation procedure was selected as a means for obtaining a dry composition comprising RNA, it is used herein for illustrative purposes only. The dry pellets comprising the target RNA obtained using the PURESCRIPT method were then either rehydrated as in previous RNA transcription and amplification methods, or used directly according to the present invention as set forth hereinbelow.

### Materials and Methods

Any material whose source is not specifically indicated was obtained from well-known commercial vendors. All aqueous solutions were prepared with RNase free water (Ambion, Texas).

### HIV-RNA Plasma

A human plasma stock comprising a specified copy number of HIV-RNA per unit volume was obtained from Boston Biomedica, Inc. An aliquot of the stock plasma was diluted with human plasma known to be negative for HIV-RNA to give plasma samples having 100, 500 and 1000 copies per mL of HIV-RNA, that were used in the RNA isolation procedure described below.

### RNA Isolation

The PURESCRIPT procedure was modified from that of the manufacturer as follows: 40µg glycogen, rather than 20µg was added to the plasma samples to aid in the precipitation of viral RNA. After isopropyl alcohol precipitation of RNA, one or two ethanol washes were performed on the RNA pellet and RNase-free water was used to rehydrate the RNA pellet in place of the rehydration solution provided by the manufacturer.

PURESCRIPT lysis buffer (500 µL) was added to a 1.5 mL microcentrifuge tube containing 100 µL of plasma sample described above. The contents were mixed for 5-10 seconds using a VORTEX mixer. The mixture was heated at 70°C for 5 min, cooled at room temperature for 2 min. To the mixture was added 200µL of PURESCRIPT protein-DNA Precipitation Solution. The contents were mixed gently by inverting the tube several times. The tube was then placed on ice for 5 min followed by centrifugation at 14,000 rpm (13,000-16,000 x g) for 5 min to form a tight pellet. The supernatant(about 750µL) was pipetted into a clean 1.5 mL tube containing 600 uL of 100% isopropanol and 2µL of a 20µg/mL aqueous solution of glycogen, placed on a rocker for 2 min to mix. It was recentrifuged at 14,000 rpm (13,000-16,000 x g) for 5 min. The supernatant was removed without disturbing the pellet. An ethanol wash was carried out by adding 1 mL of 70% ethanol to the pellet. The contents were mixed by inversion, recentrifuged at 14,000 rpm (13,000 -16,000 x g) for 2 min, the supernatant removed, and recentrifuged briefly (3-5 seconds) to bring down residue liquid that was then removed. The pellet was dried at room temperature.

### Reverse-Transcription (RT) Mix

The reverse-transcription (RT) mix was prepared just prior to use and stored on ice. The following reagents were used at the specified volumes per reaction: 3.0 µL of 50 µM Primer (Random Hexamer Primer (pd(N)6, Pharmacia, Upsala, Sweden), 5.0 µL 1st Strand 5x Buffer (Gibco,BRL Gaithersburgh, Maryland), 2.5 µL of 0.1 M dithiothreitol (DTT), 1.0 µL dNTP for a final total dNTP concentration of 0.4 mM, that is 0.1 mM each of Thiamine(T), Cytosine(C), Guanine(G), and Adenine(A) nucleosidetriphosphate, Pharmacia Product #27-2035-02),0.5 µL of RNasin (40 U/µL, Promega), and 1.0 µL M-MLV(200 units/µL, Gibco,BRL).

### PCR Amplification of cDNA

The cDNA admixture (25µL) obtained in Examples 1 and 2 described below was combined with 75µL of a PCR amplification admixture containing, 0.4 uM each of primers specific to the IPC (described below), HIV-1 LTR region, HIV-1 Pol region, HIV-2 Envelope region and HIV-2 LTR region, 25 mM Tris(hydroxymethyl)aminomethane Buffer, pH 8.5, 3 mM MgCl₂, 0.725 mM ethylenediaminetetraacetic acid (EDTA), 54 mM KCl, 3.72 mM NaCl, 40 uM DTT, 108µg/mL gelatin, 9.5% glycerol, 0.02% NP40 detergent (nonylphenoxypolyethoxyethananol), 2µg calf thymus DNA, total 1.2 mM dNTP's (0.3mM each T,A,G,C) 10 copies of linearized IPC Plasmid DNA, 16 Units of Taq Polymerase, and 55:1 Molar ratio of anti-Taq polymerase antibody as described in U.S. Patents 5,338,671 and 5,587,287.

The DNA was amplified using a PE-9600 Thermocycler (Perkin-Elmer Corp., Norwalk, CT) using the following protocol: an initial denaturation for 3 minutes at 96°C, followed by 5 cycles of denaturation at 96°C for 5 seconds and 62°C annealing and extention for 40 seconds, then 35 cycles of denaturation at 96° for 5 seconds and 62°C annealing and extention for 40 seconds.

### Detection of Amplified Product

PCR product was detected using a non-isotopic method. PCR products were biotinylated by use of 5' end biotin-labeled primers (sense strand) during amplification. Product was captured by hybridization to oligonucleotide probes covalently attached to latex particles which were deposited on the surface of a flow through membrane (SURECELL, Johnson&Johnson). The probe/product complex was reacted with a strepavidin-horseradish peroxidase conjugate which catalyzes the oxidative conversion of a dye precursor to a blue dye. The color intensity was scored visually (0= no color, 10=intense blue) by comparing the observed intensity to color standards. All visual color scores greater than 3 were considered positive.

In the DNA amplification and detection procedure, an internal positive control (IPC) was used to ensure that proper DNA amplification occurred. The IPC is a synthesized DNA strand of known length and copy number that was added directly to the PCR mix prior to amplification. Primers specific to the IPC were present in the PCR mix. A negative control was included in each experiment to ensure that product carryover did not occur. The negative control consisted of plasma known to be free of HIV nucleic acid. It was put through the same RNA isolation procedure and amplification procedures as the test samples. No false positives were observed with the negative controls, confirming the integrity of the results.

### EXAMPLE 1:

### Previous Method of RNA Isolation and RT-PCR

RNase-free water (24µL) was added to the dry pellet obtained from the PURESCRIPT sample preparation described above to rehydrated the RNA. It was mixed on a VORTEX mixer for 5-10 seconds, centrifuged briefly at 14,000 rpm and kept on ice for 5-10 min until the RNA pellet was completely dissolved.

An RNA transcription admixture was prepared by combining 13µL of RT Mix with 12µL of the rehydrated RNA in an RNase-free tube. Transfer of the rehydrated RNA to a new RNase-free tube was done using an RNase-free pipette tip in an RNase free workspace. RNA transcription was carried out by incubating the RNA transcription admixture at 42°C for 30 min. The admixture was then heated at 100°C for 5 min to destroy RT activity, chilled on ice for 1 min, and centrifuged briefly to bring down liquid that condensed in the tube. The admixture now comprising cDNA was immediately subjected to PCR amplification as described above. The cDNA admixture can be stored at or below -20°C prior to combining it with PCR amplification reagents if desired.

### Example 2:

### RNA Isolation and RT-PCR According to the Present Invention

The RNA transcription admixture was prepared by adding 25µL of the RT Mix directly to the dry RNA pellet obtained from the PURESCRIPT procedure as described above, mixed and centrifuged briefly to bring down small drops of liquid adhering to the sides and top of the tube. RNA transcription was carried out as described above, by incubating the RNA transcription admixture at 42°C for 30 min. It was then heated at 100°C for 5 min, chilled on ice for 1 min, and centrifuged briefly. The admixture now comprising cDNA was used for PCR immediately. As stated above, the cDNA admixture could have been stored until needed at or below -20°C prior to combination with PCR amplification reagents if desired. DNA amplification and product detection were carried out as described above.

### Results

### Comparison of RNA Isolation, Transcription and cDNA Amplification And Detection As in Example 1 (Previous Method) and Example 2 (Method Of The Present Invention) Using A Relatively High Copy Number RNA Target

### Replicates 1-5

The copy number of HIV-RNA in the original plasma sample was 1000 copies per mL. The RNA was isolated according to the method described above and transcribed and cDNA amplified and product detected as in Example 1. If all steps leading up to RNA transcription yield 100 percent recovery of the target RNA in the plasma sample, the target RNA would be present at 12.5 copies per 25 µL of the final RT admixture.

### Replicates 6-10

The copy number of HIV-RNA in the original plasma sample was 500 copies per mL. The RNA was isolated according to the method described above and transcribed and cDNA amplified and product detected as in Example 2. If all steps leading up to RNA transcription yield 100 percent recovery of the target RNA in the plasma sample, the target RNA would be present at 25 copies per 25 µL of the final RT admixture.

### Replicates 11-15

The copy number of HIV-RNA in the original plasma sample was 1000 copies per mL. The RNA was isolated according to the method described above and transcribed and cDNA amplified and product detected as in Example 2. If all steps leading up to RNA transcription yield 100 percent recovery of the target RNA in the plasma sample, the target RNA would be present at 100 copies per 100 µL of the final RT admixture.

### Replicates 16-20

AS replicates 6-10;however,two ethanol wash steps were used in the PURESCRIPT isolation procedure.

### Replicates 21-25

AS replicates 11-15;however,two ethanol wash steps were used in the PURESCRIPT isolation procedure.

The results are shown in Figure 1 and Table 1 below. HIV-1 LTR and HIV-1 Pol Probe scores are included in Table 1. IPC scores are not included as they were all positive (visual score greater than 3). HIV-2 Probes were not tested as the plasma was known to be negative for HIV-2.

**Table 1**

| RNA Transcription and cDNA Amplification Of Relatively High Copy Number Target RNA Previous Method vs Invention | | | |
|---|---|---|---|
| **Replicate Number** | **RT-PCR According To** | **HIV-1 LTR Probe Color Score** | **HIV-1 POL Probe Color Score** |
| 1 | Example 1 | 7 | 6.5 |
| 2 | Example 1 | 6 | 5.5 |
| 3 | Example 1 | 7.5 | 7 |
| 4 | Example 1 | 7 | 5 |
| 5 | Example 1 | 8 | 8 |
| 6 | Example 2 | 7.5 | 5 |
| 7 | Example 2 | 7.5 | 7 |
| 8 | Example 2 | 7.5 | 7.5 |
| 9 | Example 2 | 7 | 4.5 |
| 10 | Example 2 | 8 | 8 |
| 11 | Example 2 | 7.5 | 7.5 |
| 12 | Example 2 | 7 | 4 |
| 13 | Example 2 | 8 | 7.5 |
| 14 | Example 2 | 8 | 8 |
| 15 | Example 2 | 8 | 8 |
| 16 | Example 2 | 7 | 6.5 |
| 17 | Example 2 | 8 | 7.5 |
| 18 | Example 2 | 8 | 7.5 |
| 19 | Example 2 | 7 | 7 |
| 20 | Example 2 | 8 | 7.5 |
| 21 | Example 2 | 8 | 8 |
| 22 | Example 2 | 8 | 8 |
| 23 | Example 2 | 8 | 8 |
| 24 | Example 2 | 8 | 8 |
| 25 | Example 2 | 8 | 7 |

These experiments show that reverse-transcription of a relatively high copy number target RNA, subsequent cDNA amplification and product detection yielded similar results with a prior method and the method of the present invention.

### Comparison of RNA Isolation, Transcription and cDNA Amplification And Detection As in Example 1 (Previous Method) and Example 2 (Method Of The Present Invention) Using A Low Copy Number RNA Target

RNA transcription combined with cDNA amplification and product detection using a previous rehydration method and the method of the present invention was carried out to compare the overall assay sensitivity with a low copy number RNA target. HIV-1 RNA was isolated from plasma having a level of 100 copies per mL of plasma.

Samples having such low copy numbers must be determined in order to help reduce the spread of HIV infection and other infectious diseases. Thus, any increase in sensitivity of an assay allowing detection of low copy number target RNA is desirable.

### Replicates 1-9 and 20-27

The copy number of HIV-RNA in the original plasma sample was 100 copies per mL. The RNA was isolated according to the method described above and transcribed and cDNA amplified and product detected as in Example 1. If all steps leading up to RNA transcription yield 100 percent recovery of the target RNA in the plasma sample, the target RNA would be present at 1.25 copies per 25 µL of the final RT admixture.

### Replicates 10-18 and 28-36

The copy number of HIV-RNA in the original plasma sample was 100 copies per mL. The RNA was isolated according to the method described above and transcribed and cDNA amplified and product detected as in Example 2. If all steps leading up to RNA transcription yield 100 percent recovery of the target RNA in the plasma sample, the target RNA would be present at 2.5 copies per 25 µL of the final RT admixture.

RNA transcription, cDNA amplification and product detection were carried out as described. A negative control was included. The results are shown in Table 2 below. IPC scores are not shown, as they were all positive (visual scores greater than 3.0) except in the instance of replicate number 29. The lack of a positive signal for IPC with replicate number 29 suggests that the observed low score for HIV-1 LTR was artifact.

**Table 2**

| RNA Transcription and cDNA Amplification Of Relatively High Copy Number Target RNA Previous Method vs Invention | | | |
|---|---|---|---|
| **Previous Method** | | **Method of Invention** | |
| **Replicate Number** | **HIV-1 LTR Color Score** | **Replicate Number** | **HIV-1 LTR Color Score** |
| 1 | 0 | 10 | 5 |
| 2 | 6 | 11 | 8 |
| 3 | 0 | 12 | 7 |
| 4 | 0 | 13 | 4 |
| 5 | 0 | 14 | 6 |
| 6 | 0 | 15 | 7 |
| 7 | 5 | 16 | 7 |
| 8 | 7 | 17 | 5 |
| 9 | 0 | 18 | 6 |
| 19 | 7 | 28 | 8 |
| 20 | 8 | 29 | 2 |
| 21 | 5 | 30 | 7 |
| 22 | 0 | 31 | 7 |
| 23 | 7 | 32 | 0 |
| 24 | 0 | 33 | 6 |
| 25 | 0 | 34 | 0 |
| 26 | 0 | 35 | 7 |
| 27 | 7 | 36 | 8 |
| Sensitivity | 44% | Sensitivity | 83% |

The measure of sensitivity provided in Table 2 was determined by calculating the percentage of replicates positive for HIV-1 for each method.

A significant increase in sensitivity is demonstrated using the method of the instant invention: 44% overall sensitivity is observed with a previous rehydration method compared to an overall sensitivity of 83% using the method of the present invention with a low copy number RNA target.
This clearly demonstrates the significant advantage when employing the present invention, as sensitivity is one of the most important characteristics of a diagnostic test for infectious diseases. All the RNA that is isolated from a sample is made available for subsequent transcription using the method of the instant invention.

While the foregoing invention has been described in some detail for purposes of clarity and understanding, it will be clear to one skilled in the art from a reading of this disclosure that various changes in form and detail can be made.

## Claims

1. A method for reverse transcribing an RNA target comprising:
(i) isolating nucleic acids from a sample to form a dry composition comprising the target RNA; and
(ii) contacting the dry composition directly with an RNA transcription admixture comprising a primer that will anneal to said RNA target, four different nucleoside triphosphates, and an enzyme having reverse transcriptase activity, under conditions such that the RNA target is reverse transcribed to form cDNA complementary to the RNA target.

2. The method of claim 1 wherein said enzyme having reverse transcriptase activity is a reverse transcriptase or a DNA polymerase having reverse transcriptase activity.

3. The method of claim 2 wherein said enzyme having reverse transcriptase activity is a reverse transcriptase.

4. The method of claim 3 wherein said reverse transcriptase is Moloney murine leukemia virus reverse transcriptase, SUPERSCRIPT RNase H, SUPERSCRIPT II RNase H, or Avian Myeloblastosis Virus RT.

5. The method of claim 2 wherein said enzyme having reverse transcriptase activity is a DNA polymerase.

6. The method of claim 5 wherein said DNA polymerase is from Thermus thermophilus or Thermus aquaticus.

7. A method of amplyfying DNA comprising the steps of:
(i) isolating nucleic acids from a sample to form a dry composition comprising the target RNA;
(ii) contacting the dry composition directly with an RNA transcription admixture comprising a primer that will anneal to said RNA target, four different nucleoside triphosphates, and an enzyme having reverse transcriptase activity, under conditions such that the RNA target is reverse transcribed, thereby forming cDNA complementary to the RNA target; and
(iii) contacting the cDNA with
(a) at least two oligonucleotide primers that are sufficiently complementary to regions of the cDNA to hybridize thereto,
(b) at least four different nucleoside triphosphates,
(c) a thermostable DNA polymerization agent, under conditions such that the cDNA is amplified.

8. The method of claim 7 wherein said enzyme having reverse transcriptase activity is a reverse transcriptase or a DNA polymerase having reverse transcriptase activity.

9. The method of claim 8 wherein said enzyme having reverse transcriptase activity is a reverse transcriptase.

10. The method of claim 9 wherein said reverse transcriptase is Moloney murine leukemia virus reverse transcriptase, SUPERSCRIPT RNase H, SUPERSCRIPT II RNase H, or Avian Myeloblastosis Virus RT.

11. The method of claim 9 wherein said enzyme having reverse transcriptase activity is a DNA polymerase.

12. The method of claim 11 wherein said DNA polymerase is from Thermus thermophilus or Thermus aquaticus.

## Patentansprüche

1. Verfahren zum reversen Transkribieren eines RNA-Ziels ("Target"), umfassend:
(i) Isolieren von Nukleinsäuren aus einer Probe, um eine trockene Zusammensetzung, umfassend die Ziel-RNA, zu bilden; und
(ii) direktes In-Kontakt-Bringen der trockenen Zusammensetzung mit einer RNA-Transkriptionsmischung, umfassend einen Primer, der sich an das RNA-Ziel anlagern wird, vier verschiedene Nukleosidtriphosphate und ein Enzym mit reverser Transkriptase-Aktivität, unter solchen Bedingungen, daß das RNA-Ziel revers transkribiert wird, um eine cDNA zu bilden, die zu dem RNA-Ziel komplementär ist.

2. Verfahren nach Anspruch 1, wobei das Enzym mit reverser Transkriptase-Aktivität eine reverse Transkriptase oder eine DNA-Polymerase mit reverser Transkriptase-Aktivität ist.

3. Verfahren nach Anspruch 2, wobei das Enzym mit reverser Transkriptase-Aktivität eine reverse Transkriptase ist.

4. Verfahren nach Anspruch 3, wobei die reverse Transkriptase reverse Transkriptase von Moloney-murine-Leukämie-Virus, SUPERSCRIPT RNase H, SUPERSCRIPT II RNase H oder Vogel-Myeloblastose-Virus-RT ("Avian Myeloblastosis Virus RT") ist.

5. Verfahren nach Anspruch 2, wobei das Enzym mit reverser Transkriptase-Aktivität eine DNA-Polymerase ist.

6. Verfahren nach Anspruch 5, wobei die DNA-Polymerase aus Thermus thermophilus oder Thermus aquaticus ist.

7. Verfahren zum Amplifizieren von DNA, umfassend die Schritte:
(i) Isolieren von Nukleinsäure aus einer Probe, um eine trockenen Zusammensetzung, umfassend die Ziel-RNA, zu bilden;
(ii) direktes In-Kontakt-Bringen der trockenen Zusammensetzung mit einer RNA-Transkriptionsmischung, umfassend einen Primer, der sich an das RNA-Ziel anlagern wird, vier verschiedene Nukleosidtriphosphate und ein Enzym mit reverser Transkriptase-Aktivität, unter solchen Bedingungen, daß das RNA-Ziel revers transkribiert wird, wodurch eine cDNA gebildet wird, die zu dem RNA-Ziel komplementär ist;
(iii) In-Kontakt-Bringen der cDNA mit
(a) wenigstens zwei Oligonukleotid-Primern, die mit Regionen der cDNA hinreichend komplementär sind, um daran zu hybridisieren,
(b) wenigstens vier verschiedenen Nukleosidtriphosphaten;
(c) einem thermostabilen DNA-Polymerisationsmittel, unter solchen Bedingungen, daß die cDNA amplifiziert wird.

8. Verfahren nach Anspruch 7, wobei das Enzym mit reverser Transkriptase-Aktivität eine reverse Transkriptase oder eine DNA-Polymerase mit reverser Transkriptase-Aktivität ist.

9. Verfahren nach Anspruch 8, wobei das Enzym mit reverser Transkriptase-Aktivität eine reverse Transkriptase ist.

10. Verfahren nach Anspruch 9, wobei die reverse Transkriptase reverse Transkriptase von Moloney-murine-Läukemie-Virus, SUPERSCRIPT RNase H, SUPERSCRIPT II RNAse H oder Vogel-Myeloblastose-Virus-RT ist.

11. Verfahren nach Anspruch 9, wobei das Enzym mit reverser Transkriptase-Aktivität eine DNA-Polymerase ist.

12. Verfahren nach Anspruch 11, wobei die DNA-Polymerase von Thermus thermophilus oder Thermus aquaticus ist.

## Revendications

1. Méthode de transcription inverse d'un ARN cible comprenant :
(i) l'isolement d'acides nucléiques d'un échantillon pour former une composition sèche comprenant l'ARN cible ; et
(ii) la mise en contact de la composition sèche directement avec un mélange de transcription d'ARN comprenant une amorce qui s'hybridera audit ARN cible, quatre nucléosides triphosphates différents et une enzyme ayant une activité de transcriptase inverse, dans des conditions telles que l'ARN cible est soumis à une transcription inverse pour former un ADNc complémentaire à l'ARN cible.

2. Méthode selon la revendication 1, dans laquelle ladite enzyme ayant une activité de transcriptase inverse est une transcriptase inverse ou une ADN polymérase ayant une activité de transcriptase inverse.

3. Méthode selon la revendication 2, dans laquelle ladite enzyme ayant une activité de transcriptase inverse est une transcriptase inverse.

4. Méthode selon la revendication 3, dans laquelle ladite transcriptase inverse est la transcriptase inverse du virus de la leucémie murine de Moloney, la SuperScript RNase H, la SuperScript II RNase H ou la TI du virus de la myéloblastose aviaire.

5. Méthode selon la revendication 2, dans laquelle ladite enzyme ayant une activité de transcriptase inverse est une ADN polymérase.

6. Méthode selon la revendication 5, dans laquelle ladite ADN polymérase provient de Thermus thermophilus ou de Thermus aquaticus.

7. Méthode d'amplification d'ADN comprenant les étapes consistant à :
(i) isoler des acides nucléiques d'un échantillon pour former une composition sèche comprenant 'l'ARN cible;
(ii) mettre en contact la composition sèche directement avec un mélange de transcription d'ARN comprenant une amorce qui s'hybridera audit ARN cible, quatre nucléosides triphosphates différents et une enzyme ayant une activité de transcriptase inverse, dans des conditions telles que l'ARN cible est soumis à une transcription inverse, pour ainsi former un ADNc complémentaire à l'ARN cible ; et
(iii) mettre en contact l'ADNc avec
(a) au moins deux amorces d'oligonucléotide qui sont suffisamment complémentaires à des régions de l'ADNc pour s'y hybrider,
(b) au moins quatre nucléosides triphosphates différents,
(c) un agent de polymérisation d'ADN thermostable, dans des conditions telles que l'ADNc est amplifié.

8. Méthode selon la revendication 7, dans laquelle ladite enzyme ayant une activité de transcriptase inverse est une transcriptase inverse ou une ADN polymérase ayant une activité de transcriptase inverse.

9. Méthode selon la revendication 8, dans laquelle ladite enzyme ayant une activité de transcriptase inverse est une transcriptase inverse.

10. Méthode selon la revendication 9, dans laquelle ladite transcriptase inverse est la transcriptase inverse du virus de la leucémie murine de Moloney, la SuperScript RNase H, la SuperScript II RNase H ou la TI du virus de la myéloblastose aviaire.

11. Méthode selon la revendication 9, dans laquelle ladite enzyme ayant une activité de transcriptase inverse est une ADN polymérase.

12. Méthode selon la revendication 11, dans laquelle ladite ADN polymérase provient de Thermus thermophilus ou de Thermus aquaticus.
